(19) Europäisches Patentamt

European Patent Office

Office européen des brevets

(11) **EP 4 459 545 A1**

(12) # EUROPEAN PATENT APPLICATION
published in accordance with Art. 153(4) EPC

(43) Date of publication:
**06.11.2024 Bulletin 2024/45**

(21) Application number: **23765687.1**

(22) Date of filing: **03.02.2023**

(51) International Patent Classification (IPC):
**G06T 7/00** (2017.01)    **A61B 5/00** (2006.01)

(52) Cooperative Patent Classification (CPC):
Y02A 90/30

(86) International application number:
**PCT/CN2023/074338**

(87) International publication number:
**WO 2023/169108 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.03.2022 CN 202210234627**

(71) Applicant: **Chongqing Haifu Medical Technology Co., Ltd.**
**Chongqing 400714 (CN)**

(72) Inventors:
• **LIN, Tao**
  **Chongqing 400714 (CN)**

• **LI, Julong**
  **Chongqing 400714 (CN)**
• **TAN, Zhigang**
  **Chongqing 400714 (CN)**
• **PU, Lipeng**
  **Chongqing 400714 (CN)**
• **WU, Xiaobing**
  **Chongqing 400714 (CN)**

(74) Representative: **2K Patentanwälte Blasberg Kewitz & Reichel**
**Partnerschaft mbB**
**Schumannstrasse 27**
**60325 Frankfurt am Main (DE)**

(54) **TARGET REGION POSITIONING METHOD, ELECTRONIC DEVICE, AND MEDIUM**

(57) The present disclosure provides a method for positioning a target area, an electronic device, and a computer-readable storage medium. The method for positioning a target area includes: collecting, by using a camera, a target image including a skin surface area corresponding to a reactive bone, where the reactive bone is a bone with a target feature; identifying the skin surface area from the target image; determining first device coordinate information of a central position of the skin surface area in a device coordinate system; and determining second device coordinate information of a central position of a target area including a lesion in the device coordinate system based on the first device coordinate information and predetermined first positional relationship information, where the first positional relationship information is positional relationship information between the central position of the skin surface area and the central position of the target area.

FIG. 1

**Description**

TECHNICAL FIELD

Embodiments of the present disclosure relate to the technical field of smart medical care, and in particular, to a method for positioning a target area, an electronic device, and a computer-readable storage medium.

BACKGROUND

Minimally invasive/non-invasive treatment technology refers to a treatment method of accurately destroying and killing tumors by using focused ultrasound, argonhelium cryoablation, catheter intervention, radiofrequency ablation and other minimally invasive methods through image guidance. With the continuous development of medical technology, minimally invasive/non-invasive treatment is referred to as one of the most active and promising technologies in the field of comprehensive tumor treatment currently because it features small trauma, accurate curative effects, strong pertinence, rapid recovery, and the like. However, during treatment, due to the complexity of a patient's tissue, there are very high requirements on the ability of a doctor to find a lesion of the patient. Especially for an inexperienced doctor, it takes a very long time to find the lesion. This restricts many doctors from entering the field of minimally invasive/non-invasive treatment. In addition, a doctor determines whether a site observed is a lesion of the patient mainly depending on the subjective judgment of the doctor. As a result, a problem such as improper treatment position caused by a subjective judgment error of the doctor may occur. In addition, the patient's body position changes during the treatment, and the doctor further needs to rely on experience to reposition to continue the treatment, which also takes a very long time.
To reduce the workload of the doctor and reduce dependence of surgical operations on experience of the doctor, it is necessary to intelligently identify the position of the lesion of the patient. At present, a method of pasting a mark needs to be adopted in an intelligent identification technology. Pasting a mark increases discomfort of the patient, and selection of a marking material, training on a pasting position, and the like further increase the workload of a medical worker.

SUMMARY

Embodiments of the present disclosure provide a method for positioning a target area, an electronic device, and a computer-readable storage medium.
In a first aspect, an embodiment of the present disclosure provides a method for positioning a target area, including:

collecting, by using a camera, a target image including a skin surface area corresponding to a reactive bone, where the reactive bone is a bone with a target feature;
identifying the skin surface area from the target image;
determining first device coordinate information of a central position of the skin surface area in a device coordinate system; and
determining second device coordinate information of a central position of a target area including a lesion in the device coordinate system based on the first device coordinate information and predetermined first positional relationship information, where the first positional relationship information is positional relationship information between the central position of the skin surface area and the central position of the target area.

In some example embodiments, the identifying the skin surface area from the target image includes:

performing image enhancement processing on the target image; and
inputting a target image subjected to the image enhancement processing into a trained classification model to obtain first pixel coordinate information of the skin surface area in a pixel coordinate system.

In some example embodiments, before the inputting a target image subjected to the image enhancement processing into a trained classification model to obtain first pixel coordinate information of the skin surface area in a pixel coordinate system, the method further includes:

collecting a sample image including the skin surface area by using the camera;
performing image enhancement processing on the sample image; and
performing model training based on a sample image subjected to the image enhancement processing to obtain the classification model.

In some example embodiments, the determining first device coordinate information of a central position of the skin surface area in a device coordinate system includes:

determining second pixel coordinate information of the central position of the skin surface area in a pixel coordinate system;
determining camera coordinate information of the central position of the skin surface area in a camera coordinate system based on the second pixel coordinate information and a first transformation relationship, where the first transformation relationship is a transformation relationship between the pixel coordinate system and the camera coordinate system; and
determining the first device coordinate information based on the camera coordinate information and a second transformation relationship, where the second transformation relationship is a transformation relationship between the camera coordinate system and the device coordinate system.

In some example embodiments, the first positional relationship information is a difference between the first device coordinate information and fourth device coordinate information of the central position of the target area in the device coordinate system; and

the determining second device coordinate information of a central position of a target area including a lesion in the device coordinate system based on the first device coordinate information and predetermined first positional relationship information includes:
determining that the second device coordinate information is a difference between the first device coordinate information and the first positional relationship information.

In some example embodiments, before the collecting, by using a camera, a first image including a skin surface area corresponding to a reactive bone, the method further includes:
obtaining the first positional relationship information in advance based on a nuclear magnetic resonance image.
In some example embodiments, the obtaining the first positional relationship information in advance based on a nuclear magnetic resonance image includes:

determining first nuclear magnetic resonance coordinate information of the central position of the target area in a nuclear magnetic resonance coordinate system and second nuclear magnetic resonance coordinate information of a target position of the reactive bone in the nuclear magnetic resonance coordinate system based on the nuclear magnetic resonance image;
determining third nuclear magnetic resonance coordinate information of the central position of the skin surface area in the nuclear magnetic resonance coordinate system based on the second nuclear magnetic resonance coordinate information and the nuclear magnetic resonance image; and
determining the first positional relationship information based on the first nuclear magnetic resonance coordinate information and the third nuclear magnetic resonance coordinate information.

In some example embodiments, the determining the first positional relationship information based on the first nuclear magnetic resonance coordinate information and the third nuclear magnetic resonance coordinate information includes:

determining that the first positional relationship information is a difference between the first nuclear magnetic resonance coordinate information and the third nuclear magnetic resonance coordinate information; or
determining a difference between the first nuclear magnetic resonance coordinate information and the third nuclear magnetic resonance coordinate information; and determining that the first positional relationship information is a product of the difference and a third transformation relationship, where the third transformation relationship is a transformation relationship between the nuclear magnetic resonance coordinate system and the device coordinate system.

In a second aspect, an embodiment of the present disclosure provides an electronic device, including:

at least one processor; and
a memory, where at least one program is stored on the memory, and when the at least one program is executed by the at least one processor, the at least one processor implements the method for positioning a target area according to any one of the implementations described above.

In a third aspect, an embodiment of the present disclosure provides a computer-readable storage medium, where the computer-readable storage medium stores a computer program thereon, and when the program is executed by a processor, the method for positioning a target area according to any one of the implementations described above is implemented.

In the method for positioning a target area according to the embodiment of the present disclosure, a position of a lesion of a patient is intelligently identified and positioned during the surgical operation, and positioning accuracy of the position of the lesion of the patient is improved. Moreover, there is no need to paste a mark, thereby reducing the workload of a medical worker.

BRIEF DESCRIPTION OF DRAWINGS

Accompanying drawings are intended to provide a further understanding of embodiments of the present disclosure, and constitute a part of the specification. The accompanying drawings, together with the embodiments of the present disclosure, are intended to explain the present disclosure, but do not constitute a limitation on the present disclosure. Detailed example embodiments are described with reference to the accompanying drawings, in which:

FIG. 1 is a flowchart of a method for positioning a target area according to an embodiment of the present disclosure;
FIG. 2 is a schematic diagram showing transformation between a camera coordinate system and an image physical coordinate system according to an embodiment of the present disclosure; and
FIG. 3 is a block diagram of composition of an apparatus for positioning a target area according to another embodiment of the present disclosure.

DESCRIPTION OF EMBODIMENTS

To make those skilled in the art better understand the technical solutions of the present disclosure, a method for positioning a target area, an electronic device, and a computer-readable storage medium according to the present disclosure are described in detail below with reference to the accompanying drawings.

Example embodiments are described more fully hereinafter with reference to the accompanying drawings, but the example embodiments may be embodied in different forms and should not be construed as being limited to the embodiments set forth herein. Instead, these embodiments are provided so that the present disclosure is thorough and complete, and those skilled in the art fully understand the scope of the present disclosure.

The embodiments of the present disclosure and the features in the embodiments can be combined with each other when no conflict occurs.

As used herein, the term "and/or" includes any and all combinations of at least one related enumerated item.

The terms used herein are only intended to describe specific embodiments and are not intended to limit the present disclosure. As used herein, the singular forms "a/an" and "this" are also intended to include the plural forms unless the context clearly indicates otherwise. It shall be also understood that when the terms "including" and/or "made of" are used in this specification, the presence of the described feature, integer, step, operation, element and/or component is specified, but the presence or addition of at least one other feature, integer, step, operation, element, component and/or group thereof is not excluded.

Unless otherwise defined, all terms (including technical and scientific terms) used herein have the same meanings as commonly understood by those of ordinary skill in the art. It should be also understood that terms such as those defined in commonly used dictionaries should be interpreted as having meanings consistent with meanings thereof in the background of the related art and the present disclosure, and will not be interpreted as having idealized or overly formal meanings unless explicitly so defined herein.

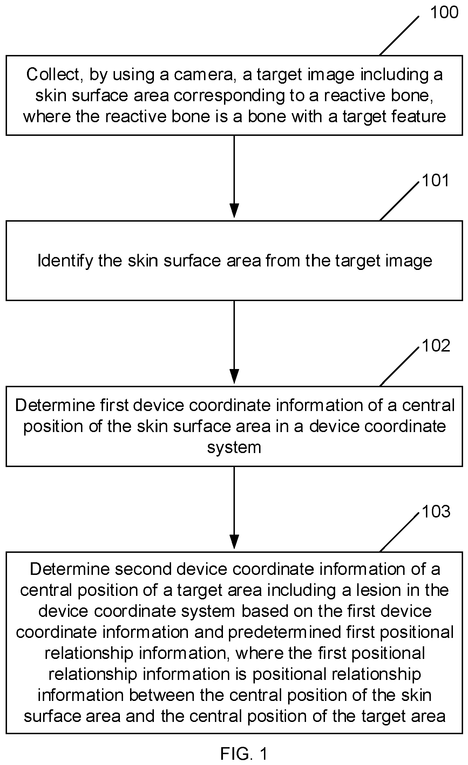

FIG. 1 is a flowchart of a method for positioning a target area according to an embodiment of the present disclosure.

In a first aspect, referring to FIG. 1, the method for positioning a target area according to an embodiment of the present disclosure includes the following steps.

Step 100: Collect, by using a camera, a target image including a skin surface area corresponding to a reactive bone, where the reactive bone is a bone with a target feature.

In some example embodiments, the camera may be any one of a monocular camera, a binocular camera, a multiocular camera, and a 3D-structured optical camera.

In some example embodiments, the reactive bone may be a bone whose spatial positional relationship with a human skin surface does not change in a natural state. For example, the reactive bone may be a nasal bone, a sacrococcygeal bone, or the like.

In some example embodiments, the skin surface area corresponding to the reactive bone refers to an area that is on the human skin surface, and has the same position as the reactive bone, but has a different depth. For example, when the reactive bone is a nasal bone, the skin surface area may be an area including the nose; or when the reactive bone is a sacrococcygeal bone, the skin surface area may be a sacrococcygeal triangular area.

Step 101: Identify the skin surface area from the target image.

In some example embodiments, the identifying the skin surface area from the target image includes: performing image enhancement processing on the target image; and inputting a target image subjected to the image enhancement processing into a trained classification model to obtain first pixel coordinate information of the skin surface area in a pixel coordinate system.

In some example embodiments, the identifying the skin surface area from the target image includes: inputting the target image into a trained classification model to obtain first pixel coordinate information of the skin surface area in a pixel coordinate system.

In some example embodiments, the pixel coordinate system is a two-dimensional coordinate system established based on the target image. An origin of the pixel coordinate system may be any point on the target image or any point on a nontarget image, for example, may be an upper left corner of the target image. One axis of the pixel coordinate system is parallel to a row of the target image, and the other axis thereof is parallel to a column of the target image; or one axis of the pixel coordinate system is parallel to the column of the target image, and the other axis thereof is parallel to the row of the target image. Pixel coordinate information of a point on the target image in the pixel coordinate system is discrete, and can only be an integer value in pixels.

In some example embodiments, because the brightness of the skin surface area in the target image is less than that of other surrounding areas, that is, the skin surface area is dark, to enhance an image contrast of the skin surface area, prevent the occurrence of excessive amplification noise caused by a close grayscale of the image and reduce an impact of lighting conditions on image features, image enhancement processing is performed on the target image. Specifically, a method familiar to those skilled in the art may be adopted to perform image enhancement processing on the target image. For example, contrast limited adaptive histogram equalization (CLAHE) may be adopted to perform image enhancement processing on the target image.

In some example embodiments, before the inputting a target image subjected to the image enhancement processing into a trained classification model to obtain first pixel coordinate information of the skin surface area in a pixel coordinate system, or before the inputting the target image into a trained classification model to obtain first pixel coordinate information of the skin surface area in a pixel coordinate system, the method further includes: collecting a sample image including the skin surface area by using the camera; performing image enhancement processing on the sample image; and performing model training based on a sample image subjected to the image enhancement processing to obtain the classification model.

In some example embodiments, before the inputting a target image subjected to the image enhancement processing into a trained classification model to obtain first pixel coordinate information of the skin surface area in a pixel coordinate system, or before the inputting the target image into a trained classification model to obtain first pixel coordinate information of the skin surface area in a pixel coordinate system, the method further includes: collecting a sample image including the skin surface area by using the camera; and performing model training based on the sample image to obtain the classification model.

In some example embodiments, a model familiar to those skilled in the art may be trained to obtain the classification model. For example, a mask region-based convolutional neural network (R-CNN) model may be trained to obtain the classification model. Specifically, a process of implementing the mask R-CNN model roughly includes: labeling a skin surface area of a sample image or a sample image subjected to image enhancement processing to generate a mask label data set; filtering and preprocessing the mask label data set, and dividing a filtered and preprocessed data set to obtain data sets with different pose image combinations; inputting the data sets with different pose image combinations into a pre-trained neural network (such as ResNet) to obtain a corresponding body surface feature map; for a region of interest (ROI) of each point in the body surface feature map, obtaining a bounding box based on the ROI; performing binary classification and bounding-box (BB) regression processing on the bounding box to filter points corresponding to part of lower score ROIs; and performing an ROI alignment operation on the remaining points in the bounding box, and classifying points after the ROI alignment operation.

Step 102: Determine first device coordinate information of a central position of the skin surface area in a device coordinate system.

In some example embodiments, the device may be any device that performs a surgical operation, such as a mechanical arm.

In some example embodiments, the device coordinate system is a three-dimensional coordinate system established based on the device.

In some example embodiments, the determining first device coordinate information of a central position of the skin surface area in a device coordinate system includes: determining second pixel coordinate information of the central position of the skin surface area in a pixel coordinate system; determining camera coordinate information of the central position of the skin surface area in a camera coordinate system based on the second pixel coordinate information and a first transformation relationship, where the first transformation relationship is a transformation relationship between the pixel coordinate system and the camera coordinate system; and determining the first device coordinate information based on the camera coordinate information and a second transformation relationship, where the second transformation relationship is a transformation relationship between the camera coordinate system and the device coordinate system.

In some example embodiments, the camera coordinate system is a three-dimensional coordinate system established based on the camera.

In some example embodiments, the first transformation relationship may be represented by a first transformation matrix.

In some example embodiments, the camera coordinate system is associated with the pixel coordinate system by using an image physical coordinate system, and the first transformation relationship may be obtained based on a transformation relationship between the camera coordinate system and the image physical coordinate system and a transformation relationship between the image physical coordinate system and the pixel coordinate system.

In some example embodiments, the transformation relationship between the camera coordinate system and the image physical coordinate system may be expressed by using a third transformation matrix, the transformation relationship between the image physical coordinate system and the pixel coordinate system may be expressed by using a fourth transformation matrix, and thus the first transformation matrix may be determined based on the third transformation matrix and the fourth transformation matrix.

In some example embodiments, the image physical coordinate system is a two-dimensional coordinate system established on an image sensor. An origin of the image physical coordinate system is an intersection of an optical axis of the camera and an imaging plane. One axis of the image physical coordinate system is parallel to a row of the image sensor, and the other axis thereof is parallel to a column of the image sensor; or one axis of the image physical coordinate system is parallel to the column of the image sensor, and the other axis thereof is parallel to a row of the image sensor. Image physical coordinate information of a point on the image sensor in the image physical coordinate system is discrete, with the length as the unit.

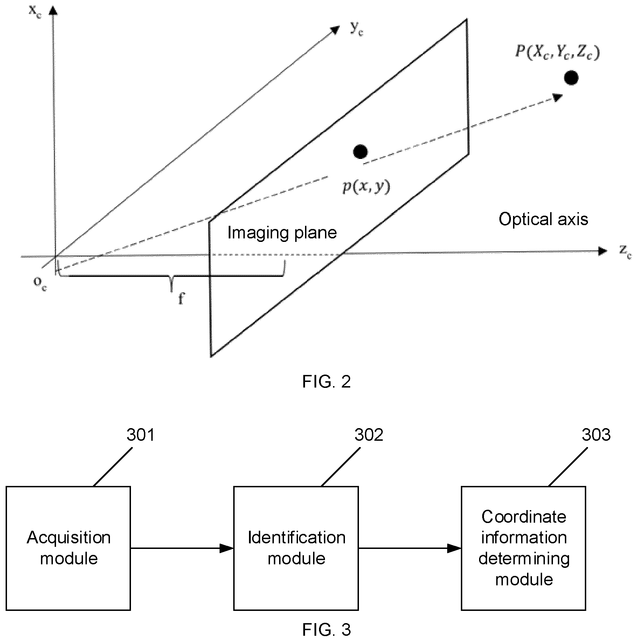

In some example embodiments, the camera coordinate system is a three-dimensional coordinate system and the image physical coordinate system is a two-dimensional coordinate system. Therefore, the third transformation matrix is a transformation matrix between the three-dimensional coordinate system and the two-dimensional coordinate system. Specifically, it is assumed that there is a point P on the skin surface area, camera coordinate information of the point P in the camera coordinate system is (Xc, Yc, Zc), and a line OcP connecting an intersection Oc of the optical axis of the camera and the imaging plane to the point P has an intersection p with the camera imaging plane, that is, a projection point of the point P on the camera imaging plane. As shown in FIG. 2, image physical coordinate information of the point p in the image physical coordinate system is (x, y)f, which is a focal length of the camera. Then it can been seen according to the principle of similar triangles that:

$$\frac{x}{f} = \frac{Xc}{Zc} \quad (1);$$

and

$$\frac{y}{f} = \frac{Yc}{Zc} \quad (2).$$

Then, the third transformation matrix may be expressed as:

$$Zc \begin{bmatrix} x \\ y \\ 1 \end{bmatrix} = \begin{bmatrix} f & 0 & 0 & 0 \\ 0 & f & 0 & 0 \\ 0 & 0 & 1 & 0 \end{bmatrix} \begin{bmatrix} Xc \\ Yc \\ Zc \\ 1 \end{bmatrix} \quad (3).$$

The fourth transformation matrix is a transformation matrix between two two-dimensional coordinate systems, that is:

$$\begin{bmatrix} u \\ v \\ 1 \end{bmatrix} = \begin{bmatrix} \alpha & 0 & u_0 \\ 0 & \beta & v_0 \\ 0 & 0 & 1 \end{bmatrix} \begin{bmatrix} x \\ y \\ 1 \end{bmatrix} \quad (4),$$

where $\alpha$ is a number of pixels in unit length in an x-axis direction, $\beta$ is a number of pixels in unit length in a y-axis direction, (u, v) is the pixel coordinate information of the point P, (x, y) is the image physical coordinate information of the point p, and $(u_0, v_0)$ is pixel coordinate information of the origin of the image physical coordinate system in the pixel coordinate system.

Then, the first transformation matrix may be expressed as:

$$Zc \times \begin{bmatrix} u \\ v \\ 1 \end{bmatrix} = \begin{bmatrix} \alpha & 0 & u_0 \\ 0 & \beta & v_0 \\ 0 & 0 & 1 \end{bmatrix} \times Zc \times \begin{bmatrix} x \\ y \\ 1 \end{bmatrix} = \begin{bmatrix} \alpha & 0 & u_0 \\ 0 & \beta & v_0 \\ 0 & 0 & 1 \end{bmatrix} \begin{bmatrix} f & 0 & 0 & 0 \\ 0 & f & 0 & 0 \\ 0 & 0 & 1 & 0 \end{bmatrix} \begin{bmatrix} Xc \\ Yc \\ Zc \\ 1 \end{bmatrix} \quad (5),$$

$$= \begin{bmatrix} \alpha & 0 & u_0 \\ 0 & \beta & v_0 \\ 0 & 0 & 1 \end{bmatrix} \begin{bmatrix} Xc \\ Yc \\ Zc \end{bmatrix} = K \times \begin{bmatrix} Xc \\ Yc \\ Zc \end{bmatrix}$$

where K is an intrinsic matrix of the camera, that is, the first transformation matrix.

In some example embodiments, the determining camera coordinate information of the central position of the skin surface area in a camera coordinate system based on the second pixel coordinate information and a first transformation relationship includes: determining the camera coordinate information according to formula (4).

In some example embodiments, the second transformation relationship may be represented by a second transformation matrix Le.

In some example embodiments, the camera coordinate system and the device coordinate system are both three-dimensional coordinate systems. Therefore, the second transformation matrix is a transformation matrix between the two three-dimensional coordinate systems, and at the same time, the skin surface area changes only in spatial position and orientation in the two three-dimensional coordinate systems, but does not change in shape. Therefore, the second transformation matrix Le may be represented by using a rotation matrix R and a translation matrix T. Specifically, it is assumed that there is a point P on the skin surface area, camera coordinate information of the point P in the camera coordinate system is (Xc, Yc, Zc), device coordinate information of the point P in the device coordinate system is (Xe, Ye, Ze), and a transformation relationship between the two pieces of coordinate information is shown in formula (5).

$$\begin{bmatrix} Xe \\ Ye \\ Ze \end{bmatrix} = R \times \begin{bmatrix} Xc \\ Yc \\ Zc \end{bmatrix} + T = \begin{bmatrix} R & T \\ O^T & 1 \end{bmatrix} \begin{bmatrix} Xc \\ Yc \\ Zc \end{bmatrix} = Le \times \begin{bmatrix} Xc \\ Yc \\ Zc \end{bmatrix} \quad (5),$$

$$R = \begin{bmatrix} r_{11} & r_{12} & r_{13} \\ r_{21} & r_{22} & r_{23} \\ r_{31} & r_{32} & r_{33} \end{bmatrix} \qquad T = \begin{bmatrix} t_x \\ t_y \\ t_z \end{bmatrix}$$

where R is a $3 \times 3$ matrix, , T is a translation vector, , and Le is an extrinsic matrix of the camera in the device coordinate system.

In some example embodiments, the determining the first device coordinate information based on the camera coordinate information and a second transformation relationship includes: determining the first device coordinate information based on formula (5).

Step 103: Determine second device coordinate information of a central position of a target area including a lesion in the device coordinate system based on the first device coordinate information and predetermined first positional relationship information, where the first positional relationship information is positional relationship information between the central position of the skin surface area and the central position of the target area.

In some example embodiments, the lesion may be a tumor, such as hysteromyoma.

In some example embodiments, the first positional relationship information is a difference between the first device coordinate information and fourth device coordinate information of the central position of the target area in the device coordinate system; and the determining second device coordinate information of a central position of a target area including a lesion in the device coordinate system based on the first device coordinate information and predetermined first positional relationship information includes: determining that the second device coordinate information is a difference between the first device coordinate information and the first positional relationship information.

In some example embodiments, the first positional relationship information is a difference between third nuclear magnetic resonance coordinate information of the central position of the skin surface area in a nuclear magnetic resonance coordinate system and first nuclear magnetic resonance coordinate information of the central position of the target area in the nuclear magnetic resonance coordinate system; and the determining second device coordinate information of a central position of a target area including a lesion in the device coordinate system based on the first device coordinate information and predetermined first positional relationship information includes: determining a difference between the first device coordinate information and the fourth device coordinate information based on a third transformation relationship and the difference between the third nuclear magnetic resonance coordinate information and the first nuclear magnetic resonance coordinate information; and determining that the second device coordinate information is a difference between the first device coordinate information and the difference between the first device coordinate information and the fourth device coordinate information.

In some example embodiments, the third transformation relationship is a transformation relationship between the nuclear magnetic resonance coordinate system and the device coordinate system that are two two-dimensional coordinate systems, and the third transformation relationship may be expressed by using a fifth transformation matrix. The third transformation matrix is similar to the second transformation matrix Le, and details are not described herein.

In some example embodiments, before the collecting, by using a camera, a first image including a skin surface area corresponding to a reactive bone, the method further includes: obtaining the first positional relationship information in advance based on a nuclear magnetic resonance image.

In some example embodiments, the obtaining the first positional relationship information in advance based on a nuclear magnetic resonance image includes: determining first nuclear magnetic resonance coordinate information of the central position of the target area in a nuclear magnetic resonance coordinate system and second nuclear magnetic resonance coordinate information of a target position of the reactive bone in the nuclear magnetic resonance coordinate system based on the nuclear magnetic resonance image; determining third nuclear magnetic resonance coordinate information of the central position of the skin surface area in the nuclear magnetic resonance coordinate system based on the second nuclear magnetic resonance coordinate information and the nuclear magnetic resonance image; and determining the first positional relationship information based on the first nuclear magnetic resonance coordinate information and the third nuclear magnetic resonance coordinate information.

In some example embodiments, the target position of the reactive bone may be a sacrococcygeal alternation part.

In some example embodiments, the central position of the skin surface area and the sacrococcygeal alternation part are at different depths but at the same position.

In some example embodiments, the determining the first positional relationship information based on the first nuclear magnetic resonance coordinate information and the third nuclear magnetic resonance coordinate information includes: determining that the first positional relationship information is a difference between the first nuclear magnetic resonance coordinate information and the third nuclear magnetic resonance coordinate information; or determining a difference between the first nuclear magnetic resonance coordinate information and the third nuclear magnetic resonance coordinate information; and determining that the first positional relationship information is a product of the difference and a third transformation relationship, where the third transformation relationship is a transformation relationship between the nuclear magnetic resonance coordinate system and the device coordinate system.

In the method for positioning a target area according to the embodiment of the present disclosure, a position of a lesion of a patient is intelligently identified and positioned during the surgical operation, and positioning accuracy of the position of the lesion of the patient is improved. Moreover, there is no need to paste a mark, thereby reducing the workload of a medical worker.

In a second aspect, another embodiment of the present disclosure provides an electronic device, including:

at least one processor; and
a memory, where at least one program is stored on the memory, and when the at least one program is executed by the at least one processor, the at least one processor implements the method for positioning a target area according to any one of the implementations described above.

The processor is a device with a data processing capability, and includes, but is not limited to, a central processing unit (CPU). The memory is a device with a data storage capability, and includes, but is not limited to, a random access memory (RAM, more specifically, for example, an SDRAM or a DDR), a read-only memory (ROM), an electrically erasable programmable read-only memory (EEPROM), and a flash memory.

In some example embodiments, the processor and the memory are connected to each other by a bus, and then are connected to other components of a computing device.

In some example embodiments, the electronic device further includes: a camera, configured to collect a target image including a skin surface area corresponding to a reactive bone, where the reactive bone is a bone with a target feature.

In a third aspect, another embodiment of the present disclosure provides a computer-readable storage medium, where the computer-readable storage medium stores a computer program thereon, and when the program is executed by a processor, the method for positioning a target area according to any one of the implementations described above is implemented.

FIG. 3 is a block diagram of composition of an apparatus for positioning a target area according to another embodiment of the present disclosure.

In a fourth aspect, another embodiment of the present disclosure provides an apparatus for positioning a target area, including: an acquisition module 301, configured to collect, by using a camera, a target image including a skin surface area corresponding to a reactive bone, where the reactive bone is a bone with a target feature; an identification module 302, configured to identify the skin surface area from the target image; and a coordinate information determining module 303, configured to determine first device coordinate information of a central position of the skin surface area in a device coordinate system, and determine second device coordinate information of a central position of a target area including a lesion in the device coordinate system based on the first device coordinate information and predetermined first positional relationship information, where the first positional relationship information is positional relationship information between the central position of the skin surface area and the central position of the target area.

In some example embodiments, the identification module 302 is specifically configured to perform image enhancement processing on the target image, and input a target image subjected to the image enhancement processing into a trained classification model to obtain first pixel coordinate information of the skin surface area in a pixel coordinate system.

In some example embodiments, the acquisition module 301 is further configured to collect a sample image including the skin surface area by using the camera; and the identification module 302 is further configured to perform image enhancement processing on the sample image; and perform model training based on a sample image subjected to the image enhancement processing to obtain the classification model.

In some example embodiments, the coordinate information determining module 303 is specifically configured to implement the "determining first device coordinate information of a central position of the skin surface area in a device coordinate system" in the following way: determining second pixel coordinate information of the central position of the skin surface area in a pixel coordinate system; determining camera coordinate information of the central position of the skin surface area in a camera coordinate system based on the second pixel coordinate information and a first transformation relationship, where the first transformation relationship is a transformation relationship between the pixel coordinate system and the camera coordinate system; and determining the first device coordinate information based on the camera coordinate information and a second transformation relationship, where the second transformation relationship is a transformation relationship between the camera coordinate system and the device coordinate system.

In some example embodiments, the first positional relationship information is a difference between the first device coordinate information and fourth device coordinate information of the central position of the target area in the device coordinate system; and the coordinate information determining module 303 is specifically configured to implement the "determining second device coordinate information of a central position of a target area including a lesion in the device coordinate system based on the first device coordinate information and predetermined first positional relationship information" in the following way: determining that the second device coordinate information is a difference between the first device coordinate information and the first positional relationship information.

In some example embodiments, the acquisition module 301 is further configured to obtain the first positional relationship information in advance based on a nuclear magnetic resonance image.

In some example embodiments, the acquisition module 301 is specifically configured to implement the "obtaining the first positional relationship information in advance based on a nuclear magnetic resonance image" in the following way: determining first nuclear magnetic resonance coordinate information of the central position of the target area in a nuclear magnetic resonance coordinate system and second nuclear magnetic resonance coordinate information of a target position of the reactive bone in the nuclear magnetic resonance coordinate system based on the nuclear magnetic resonance image; determining third nuclear magnetic resonance coordinate information of the central position of the skin surface area in the nuclear magnetic resonance coordinate system based on the second nuclear magnetic resonance coordinate information and the nuclear magnetic resonance image; and determining the first positional relationship information based on the first nuclear magnetic resonance coordinate information and the third nuclear magnetic resonance coordinate information.

In some example embodiments, the acquisition module 301 is specifically configured to implement the "determining the first positional relationship information based on the first nuclear magnetic resonance coordinate information and the third nuclear magnetic resonance coordinate information" in the following way: determining that the first positional relationship information is a difference between the first nuclear magnetic resonance coordinate information and the third nuclear magnetic resonance coordinate information; or determining a difference between the first nuclear magnetic resonance coordinate information and the third nuclear magnetic resonance coordinate information; and determining that the first positional relationship information is a product of the difference and a third transformation relationship, where the third transformation relationship is a transformation relationship between the nuclear magnetic resonance coordinate system and the device coordinate system.

The specific implementation process of the above apparatus for positioning a target area is the same as the specific implementation process of the method for positioning a target area in the aforementioned embodiment, and details are not described herein.

Those of ordinary skill in the art can understand that all or some of the steps of the method, a system and functional modules/units in the apparatus, which are disclosed above, can be implemented as software, firmware, hardware, and an appropriate combination thereof. In a hardware implementation, the division between functional modules/units mentioned in the above description does not necessarily correspond to the division of physical components. For example, a physical component may have multiple functions, or a function or step may be performed by a plurality of physical components in cooperation. Some or all of the physical components may be implemented as software executed by a processor, such as a central processing unit, a digital signal processor or a microprocessor, or as hardware, or as an integrated circuit, such as an application-specific integrated circuit. Such software may be distributed on computer-readable medium. The computer-readable medium may include a computer storage medium (or non-transitory medium) and a communication medium (or transitory medium). As is well known to those of ordinary skill in the art, the term computer storage medium includes volatile and nonvolatile, and removable and non-removable media implemented in any method or technology for storing information (such as computer-readable instructions, data structures, program modules or other data). The computer storage medium includes, but is not limited to, a RAM, a ROM, an EEPROM, a flash memory or other memories, a CD-ROM, a digital versatile disk (DVD) or other optical disk storages, magnetic boxes, magnetic tapes, magnetic disk storages or other magnetic storages, or any other medium that may be configured to store desired information and can be accessed by a computer. Furthermore, it is well known to those of ordinary skill in the art that the communication medium usually contains computer-readable instructions, data structures, program modules or other data in a modulated data signal such as a carrier wave or other transmission mechanism, and may include any information delivery medium.

Example embodiments have been disclosed herein, and although specific terms are adopted, the terms are only used and should only be interpreted in a general illustrative sense and are not used for limiting purposes. In some examples, it is obvious to those skilled in the art that, unless otherwise explicitly stated, features, characteristics, and/or elements described with reference to specific embodiments may be used separately, or may be used in combination with features, characteristics, and/or elements described with reference to other embodiments. Therefore, it should be understood by those skilled in the art that various changes in form and details can be made without departing from the scope of the present disclosure as set forth in the appended claims.

## Claims

1. A method for positioning a target area, comprising:

   collecting, by using a camera, a target image comprising a skin surface area corresponding to a reactive bone, wherein the reactive bone is a bone with a target feature;
   identifying the skin surface area from the target image;
   determining first device coordinate information of a central position of the skin surface area in a device coordinate system; and
   determining second device coordinate information of a central position of a target area comprising a lesion in the device coordinate system based on the first device coordinate information and predetermined first positional relationship information, wherein the first positional relationship information is positional relationship information between the central position of the skin surface area and the central position of the target area.

2. The method for positioning a target area according to claim 1, wherein the identifying the skin surface area from the target image comprises:

performing image enhancement processing on the target image; and
inputting a target image subjected to the image enhancement processing into a trained classification model to obtain first pixel coordinate information of the skin surface area in a pixel coordinate system.

3. The method for positioning a target area according to claim 2, wherein before the inputting a target image subjected to the image enhancement processing into a trained classification model to obtain first pixel coordinate information of the skin surface area in a pixel coordinate system, the method further comprises:

collecting a sample image comprising the skin surface area by using the camera;
performing image enhancement processing on the sample image; and
performing model training based on a sample image subjected to the image enhancement processing to obtain the classification model.

4. The method for positioning a target area according to claim 1, wherein the determining first device coordinate information of a central position of the skin surface area in a device coordinate system comprises:

determining second pixel coordinate information of the central position of the skin surface area in a pixel coordinate system;
determining camera coordinate information of the central position of the skin surface area in a camera coordinate system based on the second pixel coordinate information and a first transformation relationship, wherein the first transformation relationship is a transformation relationship between the pixel coordinate system and the camera coordinate system; and
determining the first device coordinate information based on the camera coordinate information and a second transformation relationship, wherein the second transformation relationship is a transformation relationship between the camera coordinate system and the device coordinate system.

5. The method for positioning a target area according to claim 1, wherein the first positional relationship information is a difference between the first device coordinate information and fourth device coordinate information of the central position of the target area in the device coordinate system; and

the determining second device coordinate information of a central position of a target area comprising a lesion in the device coordinate system based on the first device coordinate information and predetermined first positional relationship information comprises:
determining that the second device coordinate information is a difference between the first device coordinate information and the first positional relationship information.

6. The method for positioning a target area according to any one of claims 1 to 5, wherein before the collecting, by using a camera, a first image comprising a skin surface area corresponding to a reactive bone, the method further comprises:
obtaining the first positional relationship information in advance based on a nuclear magnetic resonance image.

7. The method for positioning a target area according to claim 6, wherein the obtaining the first positional relationship information in advance based on a nuclear magnetic resonance image comprises:

determining first nuclear magnetic resonance coordinate information of the central position of the target area in a nuclear magnetic resonance coordinate system and second nuclear magnetic resonance coordinate information of a target position of the reactive bone in the nuclear magnetic resonance coordinate system based on the nuclear magnetic resonance image;
determining third nuclear magnetic resonance coordinate information of the central position of the skin surface area in the nuclear magnetic resonance coordinate system based on the second nuclear magnetic resonance coordinate information and the nuclear magnetic resonance image; and
determining the first positional relationship information based on the first nuclear magnetic resonance coordinate information and the third nuclear magnetic resonance coordinate information.

8. The method for positioning a target area according to claim 7, wherein the determining the first positional relationship information based on the first nuclear magnetic resonance coordinate information and the third nuclear magnetic resonance coordinate information comprises:

determining that the first positional relationship information is a difference between the first nuclear magnetic resonance coordinate information and the third nuclear magnetic resonance coordinate information; or determining a difference between the first nuclear magnetic resonance coordinate information and the third nuclear magnetic resonance coordinate information; and determining that the first positional relationship information is a product of the difference and a third transformation relationship, wherein the third transformation relationship is a transformation relationship between the nuclear magnetic resonance coordinate system and the device coordinate system.

9. An electronic device, comprising:

at least one processor; and
a memory, wherein at least one program is stored on the memory, and when the at least one program is executed by the at least one processor, the at least one processor implements the method for positioning a target area according to any one of claims 1 to 8.

10. A computer-readable storage medium, wherein the computer-readable storage medium stores a computer program thereon, and when the program is executed by a processor, the method for positioning a target area according to any one of claims 1 to 8 is implemented.

Collect, by using a camera, a target image including a skin surface area corresponding to a reactive bone, where the reactive bone is a bone with a target feature — 100

↓

Identify the skin surface area from the target image — 101

↓

Determine first device coordinate information of a central position of the skin surface area in a device coordinate system — 102

↓

Determine second device coordinate information of a central position of a target area including a lesion in the device coordinate system based on the first device coordinate information and predetermined first positional relationship information, where the first positional relationship information is positional relationship information between the central position of the skin surface area and the central position of the target area — 103

FIG. 1

FIG. 2

FIG. 3

## INTERNATIONAL SEARCH REPORT

| International application No. |
| --- |
| **PCT/CN2023/074338** |

| A. | CLASSIFICATION OF SUBJECT MATTER |
| --- | --- |

G06T7/00(2017.01)i;A61B5/00(2006.01)i

According to International Patent Classification (IPC) or to both national classification and IPC

| B. | FIELDS SEARCHED |
| --- | --- |

Minimum documentation searched (classification system followed by classification symbols)

IPC: G06T, A61B,

Documentation searched other than minimum documentation to the extent that such documents are included in the fields searched

Electronic data base consulted during the international search (name of data base and, where practicable, search terms used)

CNTXT, ENTXTC, VEN, CNKI, IEEE: 鼻梁, 标定, 标记, 标签, 标识, 标志, 体表, 病灶, 无需, 不要, 定位, 反应骨, 皮肤, 固定点, 机械臂, 解剖, 人体, 设备坐标, 世界坐标, 手术, 导航, 特征, 位置, 稳定, 预设点, 转换, 坐标, 骶, bone, tag, skin, focus, disease, coordinat+, surgery, surgical navigat+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 114638798 A (CHONGQING HAIFU MEDICAL TECHNOLOGY CO., LTD.) 17 June 2022 (2022-06-17)<br>claims 1-10 | 1-10 |
| A | CN 113041519 A (CHONGQING HAIFU MEDICAL TECHNOLOGY CO., LTD.) 29 June 2021 (2021-06-29)<br>description, paragraphs [0027]-[0050] | 1-10 |
| A | CN 113397704 A (WUHAN UNITED IMAGING ZHIRONG MEDICAL TECHNOLOGY CO., LTD.) 17 September 2021 (2021-09-17)<br>entire document | 1-10 |
| A | CN 113274130 A (SHANGHAI UNIVERSITY) 20 August 2021 (2021-08-20)<br>entire document | 1-10 |
| A | CN 112258494 A (BEIJING WEHEALTH TECHNOLOGY CO., LTD.) 22 January 2021 (2021-01-22)<br>entire document | 1-10 |

☑ Further documents are listed in the continuation of Box C.　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 May 2023** | **10 May 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/074338**

**C.    DOCUMENTS CONSIDERED TO BE RELEVANT**

| Category* | Citation of document, with indication, where appropriate, of the relevant passages | Relevant to claim No. |
|---|---|---|
| A | US 2004143243 A1 (WAHRBURG, Jurgen) 22 July 2004 (2004-07-22)<br>entire document | 1-10 |

Form PCT/ISA/210 (second sheet) (July 2022)

INTERNATIONAL SEARCH REPORT

Information on patent family members

International application No.

**PCT/CN2023/074338**

| Patent document cited in search report | | | Publication date (day/month/year) | Patent family member(s) | | | Publication date (day/month/year) |
|---|---|---|---|---|---|---|---|
| CN | 114638798 | A | 17 June 2022 | None | | | |
| CN | 113041519 | A | 29 June 2021 | None | | | |
| CN | 113397704 | A | 17 September 2021 | None | | | |
| CN | 113274130 | A | 20 August 2021 | None | | | |
| CN | 112258494 | A | 22 January 2021 | None | | | |
| US | 2004143243 | A1 | 22 July 2004 | WO | 0200131 | A1 | 03 January 2002 |
| | | | | CA | 2413133 | A1 | 03 January 2002 |
| | | | | JP | 2004500958 | A | 15 January 2004 |
| | | | | EP | 1294300 | A1 | 26 March 2003 |
| | | | | KR | 20030018008 | A | 04 March 2003 |

Form PCT/ISA/210 (patent family annex) (July 2022)